(19) Europäisches Patentamt
European Patent Office
Office européen des brevets

(11) Veröffentlichungsnummer: **0 224 045**
**B1**

(12) **EUROPÄISCHE PATENTSCHRIFT**

(45) Veröffentlichungstag der Patentschrift: **18.07.90**

(51) Int. Cl.[5]: **C 08 B 37/08,** A 61 K 7/06, A 61 K 7/48

(21) Anmeldenummer: **86114785.8**

(22) Anmeldetag: **24.10.86**

# (54) Kosmetische Mittel auf der Basis von N-Hydroxypropylchitosanen, neue N-Hydroxypropyl-chitosane sowie Verfahren zu ihrer Herstellung.

(30) Priorität: **22.11.85 DE 3541305**

(43) Veröffentlichungstag der Anmeldung:
**03.06.87 Patentblatt 87/23**

(45) Bekanntmachung des Hinweises auf die Patenterteilung:
**18.07.90 Patentblatt 90/29**

(84) Benannte Vertragsstaaten:
**AT BE CH DE ES FR GB GR IT LI LU NL SE**

(56) Entgegenhaltungen:
**EP-A-0 028 126**
**EP-A-0 097 229**
**EP-A-0 193 736**

(73) Patentinhaber: **Wella Aktiengesellschaft**
**Berliner Allee 65**
**D-6100 Darmstadt (DE)**

(72) Erfinder: **Lang, Günther, Dr.**
**Auf der Roten Erde 10B**
**D-6107 Reinheim 5 (DE)**
Erfinder: **Maresch, Gerhard**
**Hölgesstrasse 19**
**D-6100 Darmstadt (DE)**
Erfinder: **Wendel, Harald**
**Grabengasse 3**
**D-6105 Ober-Ramstadt (DE)**
Erfinder: **Konrad, Eugen**
**Mecklenburger Strasse 101**
**D-6100 Darmstadt (DE)**
Erfinder: **Lenz, Hans-Rudi**
**Moltkestrasse 16**
**D-6100 Darmstadt (DE)**
Erfinder: **Titze, Hans-Jürgen**
**Pestalozzistrasse 35**
**D-6101 Gross-Bieberau (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft kosmetische Mittel zur Behandlung von Haaren oder der Haut mit einem Gehalt an neuen makromolekularen, vom Chitosan abgeleiteten Verbindungen, welche ein einer geeigneten Kosmetikgrundlage zur Anwendung kommen.

Die Erfindung betrifft weiterhin neue N-Hydroxypropylchitosane sowie Verfahren zu ihrer Herstellung.

Es ist bereits bekannt, in kosmetischen Mitteln, insbesondere für die Behandlung von Haaren, kationaktive Polymere, insbesondere Polymere, die quaternäre Ammoniumgruppen aufweisen, als Konditionierungsmittel einzusetzen. Auf Grund einer Wechselwirkung zwischen ihren Ammoniumgruppen und den anionischen Gruppen des Haares besitzen die kationaktiven Polymere eine große Affinität zur Keratinfaser.

Es wurde festgestellt, daß der Einsatz derartiger kationaktiver Polymere in solchen kosmetischen Mitteln zahlreiche Vorteile ergibt: Die Entwirrung der Haare sowie deren Behandlung wird erleichtert, und weiterhin werden dem Haar Sprungkraft und Glanzwirkung verliehen. Durch die Affinität zum Keratin neigen diese Polymere jedoch bei wiederholter Anwendung zur Ansammlung auf den Haaren, so daß diese schwerer werden, was im Endeffekt unerwünscht ist.

Weiterhin ergeben sich bei synthetischen Polymeren Probleme wegen der physiologischen Wirkung von eventuell vorhandenen Monomerspuren, die nur schwer aus dem Polymer entfernbar sind.

Man hat bereits versucht, diese vorerwähnten Nachteile dadurch zu beheben, daß wasserlösliche Salze des Chitosans, eines durch Entacetylierung von Chitin herstellbaren Polyglucosamins, in derartigen kosmetischen Mitteln Anwendung finden. In diesem Zusammenhang wird Bezug genommen auf die eigene europäische Patentschrift 0 002 506 sowie die eigene deutsche Patentschrift 26 27 419.

In gleicher Weise wie bei der Mehrzahl der kationaktiven Polymere mit quaternärer Gruppierung ergibt Chitosan ebenfalls häfig den Nachteil, daß es mit den anionaktiven oberflächenaktiven Agenzien, die üblicherweise in kosmetischen Mitteln zur Behandlung von Haaren, insbesondere in Shampoos, Anwendung finden, wenig verträglich ist. Es ist daher notwendig, das Chitosan in separaten Behandlungen, nämlich vor und/oder nach der Shampooierung zur Einwirkung zu bringen.

Das Chitosan erweist sich weiterhin in neutralem und alkalischem Medium als praktisch unlöslich, so daß seine Anwendung beispielsweise in alkalischen Dauerwellmitteln oder Haarfärbemitteln nicht möglich ist.

Durch Einsatz von Glycidylchitosanen anstelle von Chitosansalzen gemäß DE—OS 32 23 423 lassen sich die vorstehend erwähnten Nachteile vermeiden. Die Umsetzung von Chitosan mit Glycid ist jedoch sehr kostenintensiv, da Glycid ein teurer, nicht großtechnisch hergestellter, Rohstoff ist.

Aufgabe der Erfindung ist es daher, kostengünstigere Mittel zur Verfügung zu stellen, mit denen sich die oben aufgeführten Nachteile vermeiden lassen.

Bei Fortführung der Untersuchungen mit Chitosan und den davon abgeleiteten Verbindungen wurde nunmehr gefunden, daß bestimmte Chitosan-Derivate, und zwar speziell N-Hydroxypropyl-chitosane, die vorstehend aufgeführten Nachteile ebenfalls nicht aufweisen und zudem wesentlich kostengünstiger als die bisher bekannten Glycidyl-chitosane erhältlich sind.

Im Gegensatz zu synthetischen Polymeren mit endlichen Restmonomerengehalten sind diese N-Hydroxypropyl-chitosane physiologisch unbedenklich und biologisch abbaubar. Aufgrund ihrer Film- und Löseeigenschaften, ihrer Verdickerwirkung und Aniontensidverträglichkeit können sie nicht nur als neue, interessante Rohstoffe für Kosmetika, sondern ebenso in der Pharmazi, als Flokkungs- und Verdickungsmittel in der Abwasseraufbereitung, als Appretur- und Schlichtemittel in der Textilindustrie sowie bei der Papierherstellung Anwendung finden.

Mit N-Hydroxypropyl-chitosanen oder ihren Salzen mit organischen oder anorganischen Säuren lassen sich somit kosmetische Mittel zur Behandlung von Haaren oder der Haut herstellen, die sich durch überraschend vorteilhafte Eigenshaften auszeichnen und dadurch gekennzeichnet sind, daß sie in einer geeigneten Kosmetikgrundlage ein N-Hydroxypropyl-chitosan, bestehend aus

a) 4 bis 40 Molprozent Einheiten der Formel (I)

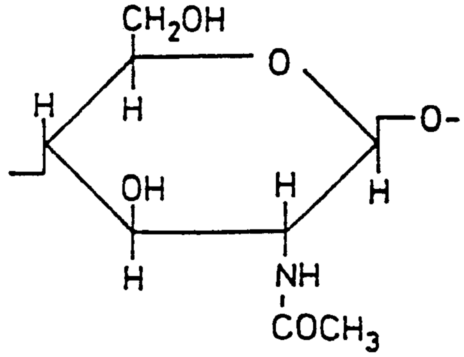

(I)

b) 60 bis 96 Molprozent Einheiten der Formel (II)

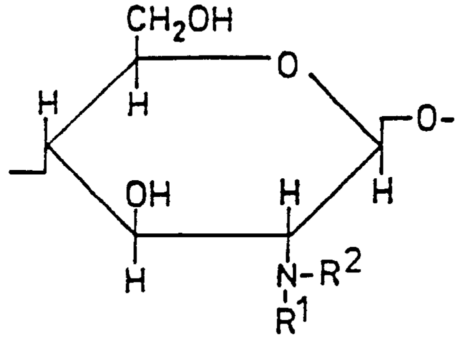

(II),

worin $R^1$ und $R^2$ gleich oder verschieden sind und Wasserstoff oder die Gruppe

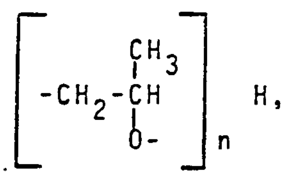

mit n gleich eine ganze Zahl von 1 bis 5, bedeuten, unter der Bedingung, daß bei mindestens 50 Prozent der Einheiten der Formel II $R^1$ und $R^2$ nicht gleitzeitig Wasserstoff darstellen, oder dessen lösliche Salze mit organischen oder anorganischen Säuren, enthalten.

Die erfindungsgemäßen N-Hydroxypropyl-chitosan enthaltenden Mittel eignen sich ganz allgemein zur Behandlung der Haut und/oder der Haare. Sie können beispielsweise vorliegen als Haar- und/oder Körperwaschmittel, Tönungsshampoos, Frisiercremes, Frisierlotionen, Fönlotionen, Mittel zur Festigung der Frisur, Waschlotionen, Haarkuren, Mittel gegen Kopfschuppen, Mittel zur permanenten Haarverformung, Mittel zur Färbung oder Entfärbung von Haaren, Mittel zum Auftragen vor oder nach der Haarfärbung und als kosmetische Mittel zur Pflege, zum Schutz oder zur Reinigung der Haut wie Gesichtwässer, Rasierwässer, Feuchthaltecremes, Coldcremes, Körperlotionen, Sonnenschutzmittel oder auch Make-up-Präparate wie Schminkcremes und Rouges.

Der Gehalt der erfindungsgemäßen kosmetischen Mittel an N-Hydroxypropyl-chitosan liegt hierbei zweckmäßig bei 0,05 bis 10 Gewichtprozent, vorzugsweise bei 0,05 bis 3,0 Gewichtsprozent.

Die kosmetischen Mittel gemäß der vorliegenden Erfindung können zusätzlich zu dem neuen Wirkstoff N-Hydroxypropyl-chitosan zur Herstellung einer Kosmetikgrundlage alle diejenigen Bestandteile enthalten, die in Haar- und Hautbehandlungsmitteln überlicherweise eingesetzt werden, insbesondere anionische, kationische, amphotere, zwitterionische oder nichtionische oberflächenaktive Tenside, Schaumsynergisten, Stabilisatoren, Sequestriermittel, Pigmente, Verdicker, Emulgatoren, Pufferstoffe, Konservierungsmittel, Farbstoffe, Parfümöle, bekannte kosmetische Polymer wie anionische, nichtionische, kationische oder amphotere Polymere, Naturstoffe, kosmetische Öle, Fettalkohole, Wachse, Schaumstabilisatoren, Wirkstoffe gegen Kopfschuppen, Reduktionsmittel und Treibgase.

Die erfindungsgemäßen kosmetischen Mittel weisen in bevorzugter Weise einen pH-Wert von 2 bis 11 auf und können in Form wäßriger, alkoholischer oder wäßrigalkoholischer Zubereitungen, insbesondere als Lösungen, als Cremes, als Gele, als Dispersionen oder als Emulsionen vorliegen. Ebenfalls ist es möglich, diese Mittel mit Hilfe eines Zerstäubers beziehungsweise anderer geeigneter Sprühvorrichtungen oder im Gemisch mit üblichen unter Druck verflüssigen Treibmitteln als Aerosolspray (zum Beispiel Aersolhaarspray) oder Aerosolschaum aus einem Druckbehälter zu entnehmen.

Wenn es sich bei den erfindungsgemäßen kosmetischen Mitteln um Mittel zur Festigung der Frisur, wie flüssige Haarfestiger oder Haarsprays, handelt, dan liegen sie üblicherweise als wäßrige, alkoholische oder wäßrig-alkoholische Lösungen vor, die durch einen Gehalt an N-Hydroxypropyl-chitosan bestehend aus Einheiten der vorstehend gennanten Formeln I und II oder dessen löslichen Salzen mit organischen oder anorganischen Säuren gekennzeichnet sind. Hierbei kann das N-Hydroxypropyl-chitosan selbst als filmbildendes oder festigendes Harz eingesetzt werden; es können jedoch auch zusätzlich andere filmbildende natürliche oder synthetische kosmetische Polymere in dem erfindungsgemäßen Haarfestigungsmittel enthalten sein. Als natürliche Polymere kommen beispielsweise Schellack, Alginate, Gelatine, Pektine und Cellulosederivate in Betracht. Von den synthetischen Polymeren finden zum Beispiel Polyvinylpyrrolidon, Polyvinylacetat, Polyacrylverbindungen, wie beispielsweise Acrylsäure- oder Methacrylsäurepolymerisate, basische Polymerisate von Estern aus Acrylsäure oder Methacrylsäure mit Aminoalkoholen oder die Salze oder Quaternisierungsprodukte dieser basischen Polymerisate, Polyacrylnitril sowie Co- oder Terpolymerisate aus derartigen Verbindungen, beispielsweise Polyvinylpyrrolidon-Vinylacetat, Verwendung.

Die Mittel weisen dann insbesondere eine pH-Wert zwischen 6 und 8 auf. Solche Mittel zur Festigung der Frisur enthalten überlicherweise der filmbildenden Polymere in einer Gesamtmenge von etwa 0,05 bis 3,0 Gewichtsprozent. Enthalten die Mittel neben dem hier beschriebenen N-Hydroxypropyl-chitosan aus Einheiten der oben genannten Formeln I und II noch andere filmbildende Polymere, so reduziert sich der Gehalt an N-Hydroxypropyl-chitosan entsprechend.

Als Alkohole kommen insbesondere die für kosmetische Zwecke üblicherweise verwendeten niedren Alkohole mit 1 bis 4 Kohlenstoffatomen, wie zum Beispiel Ethanol und Isopropanol, in Betracht.

Wenn die Mittel zur Festigung der Frisur in Form von Aerosolpräparaten vorliegen, welche aus einem Druckbehälter versprüht werden, so enthalten sie in der Kosmetikgrundlage etwa 10 bis 60 Gewichtsprozent eines Treibmittels. Als Treibmittel können Chlorfluoralkane, wie zum Beispiel $CCl_3F$, $CCl_2F_2$, $C_2Cl_3F_3$, $(CCl_2F)_2$, $CHCl_2F$ und $(CClF_2)_2$, leichtflüchtige Kohlenwasserstoffe, wie zum Beispiel n-Butan und n-Propan, oder auch Dimethylether, Kohlendioxid, Distickstoffmonoxid, Stickstoff, Methylenchlorid und 1,1,1-Trichlorethan, Verwendung finden.

Die erfindungsgemäßen Mittel zur Festigung der Frisur können weiterhin die für solche Mittel üblichen Zusätze, wie beispielsweise Parfümöle, Bakterizide oder Fungizide, kämmbarkeitsverbessernde Substanzen und Modifiziermittel, wie zum Beispiel Siliconöl oder Weichmacher, wie beispielsweise Isopropylmyristat, Phthalsäurediethylester und Diethylstearat, enthalten.

Die erfindungsgemäßen Mittel zur Festigung der Frisur können gegebenenfalls durch einem Gehalt an kosmetischen Farbstoffen das Haar gleichzeitig färben oder tönen. Derartige Präparate sind unter anderem als Farbfestiger oder Tönungsfestiger im Handel bekannt. Sie enthalten zusätzlich übliche für Haarfestiger bekannte, direkt auf das Haar aufziehende kosmetische Farbstoffe, wie beispielsweise aromatische Nitrofarbstoffe (zum Beispiel 1,4-Diamino-2-nitro-benzol, Pikraminsäure, 1-Hydroxy-2-amino-4-nitrobenzol und 1,4-Bis-[(2-hydroxyethyl)-amino]-2-nitro-5-chlor-benzol), Azofarbstoffe (zum Beispiel C.I. 14 805—Acid Brown 4), Anthrachinonfarbstoffe (zum Beispiel C.I. 61 105—Disperse Violet 4) und Triphenylmethanfarbstoffe (zum Beispiel C.I. 42 535—Basic Violet 1), wobei die Farbstoffe dieser Klassen je nach der Art ihrer Substituenten sauren, nichtionogenen oder basischen Charakter haben können. Ihre Gesamtkonzentration in diesen Präparaten beträgt überlicherweise etwa 0,01 bis 2,0 Gewichtsprozent.

Die erfindungsgemäßen Mittel zur Festigung der Frisur weisen bei gleich guter Festigung des Haares gegenüber üblichen Mittel eine besonders gute Kämmbarkeit und einen guten Griff des Haares im nassen Zustand sowie einen besonders angenehmen Griff des Haares im getrockneten Zustand auf.

Wenn die erfindungsgemäßen Mittel Haarwaschmittel darstellen, liegen sie in Form wäßriger Lösungen oder Emulsionen vor und enthalten neben dem N-Hydroxypropyl-chitosan zumindest ein anionisches, kationisches, nicht-ionisches oder amphoteres Tensid.

In diesen Haarwaschmitteln beträgt die Konzentration des Tensides im allgemeinen etwa 3 bis 50 Gewichtsprozent, vorzugsweise 3 bis 20 Gewichtsprozent, wobei der pH-Wert im allgemeinen zwischen 3 und 9 und vorzugsweise zwischen 4 und 7 liegt.

Die erfindungsgemäßen Mittel, die in Form von Haarwaschmitteln vorliegen, enthalten im allgemeinen verschiedene Zusatzstoffe, insbesondere Parfüms, Konservierungsstoffe, Verdicker, Schaumstabilisatoren, Puffersubstanzen, kosmetische Harze, Pigmente und Farbstoffe.

Unter den Schaumstabilisatoren können die Fettamide und insbesondere die Mono- oder Diethanolamide von Kokosfettsäuren, Lauryl- oder Ölsäuremono- oder -diethanolamid, die zweckmäßigerweise in Mengen von 1 bis 10 und vorzugsweise von 1 bis Gewichtsprozent Verwendung finden, angeführt werden.

Unter den Verdickern können insbesondere Acrylpolymere und Cellulosederivate, wie zum Beispiel Carboxymethylcellulose, Hydroxypropylmethylcellulose und Hydroxyethylcellulose, angeführt werden. Die Verdicker liegen im allgemeinen in einem Anteil von 0,1 bis 5 Gewichtsprozent vor.

Unter den Tensiden oder oberflächenaktiven Agenzien, die in Kombination mit den neuen N-Hydroxypropyl-chitosan verwendet werden, können beispielsweise die folgenden genannt werden:

a) die anionischen oberflächenaktiven Agenzien, wie beispielsweise die Alkali-, Erdalkali-, Ammonium- oder Alkanolaminsalze von Alkansulfonaten, Alkylsulfaten und Alkylethersulfaten, die $C_{12}$ bis $C_{18}$-Alkyl- und insbesondere $C_{12}$ bis $C_{14}$-Alkyl-Sulfatnatriumsalze oder -Triethanolaminsalze, die Natrium- oder Triethanolaminsalze von Lauryl- oder Tetradecylethersulfaten, das Dinatriumsalz des Sulfosuccinhalbesters von Alkanolamiden, die Seifen und die Polyethercarbonsäuren;

b) die nichtionischen oberflächenaktiven Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl-, Oleyl und Stearylalkohol, allein oder im Gemisch; die Fettalkohole von oxethyliertem Lanolin oder oxethyliertes Lanolin; Polyglycerylether von gesättigten oder ungesättigten Fettalkoholen und Alkylphenolen mit 8 bis 30 Kohlenstoffatomen im Alkylrest und 1 bis 10 Glycerylheiten im Molekül; Fettsäurealkanolamide sowie oxethylierte Sorbitanfettsäureester;

c) die kationischen oberflächenaktiven Agenzien, wie beispielsweise das Dilauryldimethylammoniumchlorid, die Chloride oder Bromide von Alkyldimethylbenzylammonium, die Alkyltrimethylammoniumsalze, beispielsweise Cetyltrimethylammoniumchlorid oder -bromid, Tetradecyltrimethylammoniumchlorid oder -bromid, die Alkyldimethylhydroxymethylammoniumchloride oder -bromide, die Dalkyldimethylammoniumchloride oder -bromide, Alkylpyridiniumsalze, beispielsweise Lauryl- oder Cetylpyridiniumchlorid, die Alkylamidethyltrimethylammoniumethersulfate, Imidazolinderivate,

Verbindungen mit kationischem Charakter wie Aminoxide, beispielsweise Alkyldimethylaminoxide oder Alkylaminoethyldimethylaminoxide;

d) amphoteren oder zwitterionischen oberflächenaktiven Agenzien wie beispielsweise die Carboxylderivate von Imidazol, die N-Alkylbetaine, die N-Alkylaminobetaine, die N-Alkylsulfobetaine, die N-Alkylaminopropionate, die Alkyldimethylammoniumacetate, die $C_{12}$ bis $C_{18}$-Alkyldimethylcarboxymethylammoniumsalze sowie die Fettsäurealkylamidobetaine, beispielsweise Dimethyl-carboxymethylenpropylenamido-stearat-betain.

Die erfindungsgemäßen kosmetischen Mittel können auch Cremes oder Lotionen zur Verwendung als Haarkur oder Hautpflegemittel darstellen. Sie liegen dann meist in Form von Öl-in-Wasser- oder Wasser-in-Öl-Emulsionen oder -Suspensionen vor und enthalten zusätzlich zu den neuen N-Hydroxypropylchitosanen kationische, nichtionogene, amphotere oder anionische Emulgatoren sowie als Bestandteil der Ölphase zum Beispiel Fettalkohole, Fettsäureester oder -amide, weiterhin Parfümöle, Vaseline, Wollfettalkohol oder feste beziehungsweise flüssige Paraffine.

Wenn die erfindungsgemäßen Mittel Haartönungs- oder Haarfärbemittel darstellen, so liegen sie ebenfalls bevorzugt in Form von Cremes oder Lotionen vor und enthalten zusätzlich übliche Haarfarbstoffe aus der Gruppe der aromatischen Nitrofarbstoffe, Azofarbstoffe, Anthrachinonfarbstoffe, Triphenylmethanfarbstoffe oder auch Oxidationsfarbstoffe beispielsweise Resorcin und aromatische Diamine oder Aminophenole. Weiterhin können diesel Mittel gegebenenfalls Alkalisierungsmittel, Antioxidantien sowie weitere für solche Mittel übliche kosmetische Zusätze und Hilfsstoffe enthalten.

Die erfindungsgemäßen Mittel können auch Dauerverformungsmittel oder Fixiermittel für Haare darstellen. Sie enthalten dann zusätzlich zu den genannten N-Hydroxypropyl-chitosanen Reduktionsmittel, wie zum Beispiel Thioglykolsäure, Thiomilchsäure und Ammoniumsulfit, oder Oxidationsmittel, wie zum Beispiel Hydrogenperoxid oder Natriumbromat, sowie gegebenenfalls Alkalisierungsagenzien oder Peroxidstabilisatoren, zum Beispiel Phosphorsäure und andere kosmetische Hilfsstoffe und Zusatzstoffe, wie beispielsweise Parfümöle, Riechstoffe, Pflegestoffe und Farbstoffe.

Wie bereits erwähnt wurde, können die erfindungsgemäßen kosmetischen Mittel auch zur Behandlung der Haut verwendet werden.

In der Tat erleichtern diese kosmetischen Mittel die Befeuchtung der Haut, verhindern das Austrocknen und verleihen der Haut eine hervorragende Weichheit im Griff.

Die erfindungsgemäßen kosmetischen Mittel liegen hierzu vorzugsweise in Form von Cremes, Gelen, Emulsionen oder wäßrigen, alkoholischen oder wäßrig-alkoholischen Lösungen vor, die das N-Hydroxypropyl-chitosan in einer Konzentration von 0,1 bis 10 Gewichtsprozent und vorzugsweise von 0,2 bis 6 Gewichtsprozent enthalten.

Die im allgemeinen in diesen Kosmetikzubereitungen enthaltenen Hilfsstoffe sind beispielsweise Duftstoffe, Farbstoffe, Konservierungsmittel, Verdickungsmittel, Sequestiermittel, Emulgiermittel, Sonnenschutzfilter und ähnliche.

Diese Zubereitungen für die Hautbehandlung liegen insbesondere in Form von Cremes oder Lotionen zur Pflege der Hände oder des Gesichts oder in Form von Sonnenschutzcremes, gefärbten Cremes, Abschminkmilchprodukten, Schaumbad- und Duschbad-Präparaten oder auch in Form von Desodorierzubereitungen vor.

Diese Zubereitungen werden under Anwendung klassischer Verfahrensweisen hergestellt. Beispielsweise kann man zur Bildung einer Creme eine wäßrige Phase, die das erfindungsgemäße Chitosanderivat und gegebenenfalls andere Bestandteile oer Hilfsstoffe gelöst enthält, und eine ölige Phase emulgieren. Für die ölige Phase kann man verschiedenartige Verbindungen verwenden, beispielsweise Paraffinöl, Vaselinöl, Süßmandelöl, Avocadoöl, Olivenöl, Fettsäureester, wie zum Beispiel Glycerylmonostearate, Ethylpalmitat und Isopropylpalmitat, oder Alkylmyristate, wie zum Beispiel Propylmyristat, Butylmyristat und Cetylmyristat. Man kann sich auch mit Fettsäurealkoholen, beispielsweise Stearyl- oder Cetylalkohol, oder Wachsen, beispielsweise Bienenwachs oder Wollwachs versetzen.

Die N-Hydroxypropyl-chitosanderivate können in diesen Kosmetikzubereitungen für die Hautpflege sowohl als Hauptwirkstoff als auch als Hilfstoff enthalten sein.

Die in den erfindingsgemäßen kosmetischen Mitteln enthaltenen neuen Chitosan-Derivate leiten sich von Chitosan ab, einem Material, das durch Entacetylierung von Chitin, einem natürlich vorkommenden Acetylglucosamin, erhalten wird.

Das Chitosan ist im neutralen und alkalischen Medium unlöslich, es bildet jedoch auf Grund seiner chemischen Natur im sauren Medium mit organischen und anorganischen Säuren Salze. Diese finden beispielsweise in der Papier- und Textilindustrie als Additive Verwendung. Weiterhin werden sie als Koagulanzien für Suspenionen, als Chelatbildner für Schermetallionen sowie in der Medizin und in der Kosmetik benutzt (siehe in diesem Zusammenhang die Veröffentlichung von Muzarelli: "Chitin", Pergamon Press, 1977).

Es sind bereits einige wasserlösliche Chitosanderivate bekannt, beispielsweise Carboxymethylchitosan (siehe Nud'ga, Plisko und Danilov, Zhur. Obsh. Khim. 43, No. 12, Seite 2752 bis 2756 (1973); SU—PS 325 234; sowie Okimasu, Nippon Nogei Kagaku Kaishi 32, Seite 383 bis 389 und 471 bis 473 (1958)) oder Sulfoethylchitosan (siehe Nud'ga, Plisko und Danilov, Zhur. Prikl, Khim. 47, No. 4, Seite 872 bis 874 (1974)). Diese wasserlöslichen Chitosanderivate sind jedoch entweder in ihrem ionischen Charakter verändert oder aber physiologisch bedenklich.

Hydroxyethylchitosan (Glykolchitosan) wurde von SENJU and OKIMASI (Nippon Nogei Kagaku Kaishi 23, Seite 432 bis 537 (1950)) bei der Glykolierung von Chitin in Gegenwart starken Alkalis durch gleichzeitige Entacetylierung erhalten.

Aufgrund niedriger Substitutionsgrade beziehungsweise Quervernetzung wasserunlösliche Hydroxyalkylderivate des Chitosans, deren stark wasserabsorbierende Eigenschaften anwendungstechnisch von Interesse sind, werden in der JP—PS 54—11 955 von 1979 erwähnt.

Schließlich beschreibt die JP—PS 57—180 602 von 1982 die Synthese wasserlöslicher Chitosanderivate, die durch Reaktion von Alkylenoxiden mit Chitosan an Anwesenheit von Alkali in einem Gemisch von Wasser und einem organischen Lösungsmittel erhalten werden.

All diesen mehr oder weniger wasserlöslichen Derivaten liegt die Umsetzung des Chitosans mit Alkylierungsmitteln in Gegenwart starken Alkalis zugrunde, die unter den gewählten Rekationsbedingungen ausschließlich oder überwiegend eine O-Substitution zur Folge hat. Die zur O-Alkylierung notwendige Anwesenheit von Alkali bestimmt jedoch nicht nur den Substitutionsort, sondern bewirkt darüberhinaus, insbesondere bei höheren Temperaturen, einen Abbau der Polymerkette. Desweiteren stellen die nach der Reaktion durch Neutralisation des überschüssigen Alkalis entstehenden Salze Nebenprodukte dar, die weitere Reinigungsschritte notwendig machen.

In Gegensatz hierzu berichtet die DE—OS 32 23 423 sowie die EP—OS 0 097 229 von wasserlöslichen N-substituierten Chitosanderivaten, die in bevorzugter Weise durch die Reaktion einer wäßrigen Dispersion von Chitosan mit Glycid erhalten werden. Die rasche Hydrolyse des Glycids in Gegenwart von Wasser, sein hoher Preis und die Tatsache, daß Glycid nicht großtechnisch hergestellt wird, verteuern jedoch das Verfahren zur Herstellung dieser Derivate.

Überträgt man die in der DE—OS 32 23 423 angegebenen, bevorzugt gewählten Reaktionsbedingungen auf die Umsetzung von Chitosan mit Propylenoxid, so erhält man keine wasserlöslichen Derivate.

Überraschenderweise wurde nunmehr gefunden, daß sich bei Verwendung von Gemischen aus Wasser und organischen Lösungsmitteln Chitosan auf einfache und preiswerte Weise mit Propylenoxid zu Hydroxypropylderivaten mit besonders vorteilhaften Film- und Löseeigenschaften umwandeln läßt.

In Abwesenheit basischer Katalysatoren kommt es hierbei zur Substitution der freien Aminogruppen, was durch Ermittlung des primären Amin-Stickstoffs nach van Slyke (siehe K. H. Bauer und H. Moll, "Die organische Analyse", 2. Auflage, Seite 170 bis 172, Akademische Verlagsgesellschaft Geest & Portig KG, Leipzig 1950 und H. Roth, E. v. Hulle u. a. in "Analytische Methoden", Seite 674 bis 676, Georg Thieme Verlag, Stuttgart (1953) sowie durch $^{13}C$-NMR-Spektren bestätigt wird.

Gegenstand der vorliegenden Erfindung sind daher ferner sowohl wasser- als auch alkohollösliche, vom Chitosan abgeleitete N-Hydroxypropylchitosane und deren lösliche Salze, bestehend aus

a) 4 bis 40 Molprozent Einheiten der Formel I

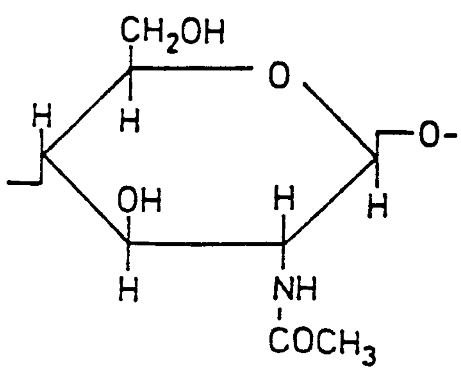

(I)

b) 60 bis 96 Molprozent Einheiten der Formel II

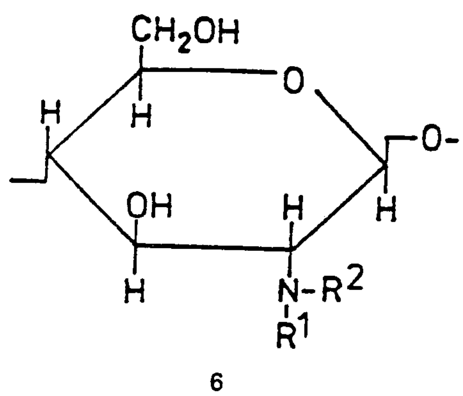

(II),

wobei $R^1$ und $R^2$ gleich oder verschieden sind und entweder Wasserstoff oder die Gruppe

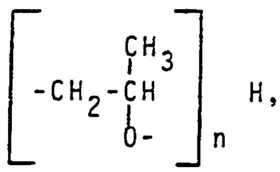

mit n gleich eine ganze Zahl von 1 bis 5, bedeuten, unter der Bedingung, daß bei mindestens 50 Prozent der Einheiten der Formel II $R^1$ und $R^2$ nicht gleichzeitig Wasserstoff darstellen.

Die neuen Chitosan-Derivate werden erfindungsgemäß erhalten, indem man Chitosan (zu 60 bis 96 Prozent entacetyliertes Chitin) oder seine Salze bei Temperaturen zwischen 20 und 120°C, vorzugsweise zwischen 40 und 100°C, drucklos im offenen Reaktor oder unter Druck im Autoklaven, mit Propylenoxid über einen Zeitraum von 3 bis 72 Stunden, vorzugsweise 6 bis 48 Stunden, umsetzt.

Man führt die Reaktion in einer Dispersion bestehend aus Wasser und einem organischen Lösungsmittel durch. In bevorzugter Weise erfolgt die Umsetzung in Anwesenheit saurer Katalysatoren, wie zum Beispiel Salzsäure, in einer Dispersion aus überschüssigem Propylenoxid und Wasser. Das molare Verhältnis von Chitosan zu Propylenoxid wählt man zwischen 1:3 und 1:15.

Nach beendeter Reaktion entfernt man das überschüssige Alkylierungsmittel, trennt eventuell vorhandene unlösliche Anteile aus de Lösungen des Chitosanderivates durch Filtration ab, neutralisiert gegebenenfalls, engt am Rotationsverdampfer ein und fällt die Chitosanderivate unmittelbar oder nach der Dialyse in Aceton aus.

Die Salze der erfindungsgemäßen N-Hydroxypropyl-chitosane können durch Neutralisierung der Aminogruppen der N-Hydroxypropyl-chitosane mit anorganischen oder organischen Säuren erhalten werden. Gemäß der vorliegenden Erfindung sind jedoch nur solche Salze verwendbar, die in Wasser löslich sind. Geeignete Salze sind zum Beispiel solche, die mit Salzsäure, Glykolsäure, Milchsäure, Ameisensäure, Zitronensäure oder Essigsäure gebildet werden.

Die nachfolgenden Beispiele sollen den Gegenstand der Erfindung näher erläutern, ohne diesen hierauf zu beschränken.

## Herstellungsbeispiele

### Beispiel 1

50 g (0,31 mol) niedermolekulares, gemahlenes Chitosan mit einer Grenzviskositätszahl (η) von 160 ml/g, und einem Entacetylierungsgrad von 90 Prozent werden zusammen mit 100 ml destillierten Wasser, 100 ml Ethanol und 209,08 g (3,6 mol) Propylenoxid in den Autoklaven eingebracht. Die gewählte Reaktionstemperatur von 100°C führt zu einem Anfangsdruck von etwa 3,5 bar, der nach 12-stündiger Reaktionszeit auf einen Wert von etwa 0,5 bar absinkt. Nach beendeter Reaktion entfernt man das überschüssige Alkylierungsmittel durch Hindurchleiten von Stickstoff oder durch kurzzeitiges Erwärmen zum Sieden, nachdem man das hochviskose Umsetzungsprodukt mit Ethanol/Wasser 1:1 verdünnt hat.

Nach Druckfiltration zur Entfernung unumgesetzter Anteile engt man das Filtrat am Rotationsverdampfer ein und fällt anschließend in der 8 bis 10-fachen Menge Aceton aus.

Um eingeschlossene Propylenglykole zu entfernen, wird das gefällte Derivat mittels eines Ultra-Turax fein dispergiert.

Der Niederschlag wird auf einer Glassinternutsche gesammelt, gründlich mit Aceton gewaschen und bei 50°C im Vakuumtrockenschrank getrocknet.

Es werden 74 g N-Hydroxypropyl-chitosan erhalten.

Kenndaten:

| | |
|---|---|
| Grenzviskositätszahl (η) | = 62 ml/g |
| Substitutionsgrad Hydroxypropyl | = 1,7 |
| Pendelhärte | = 193 sec |
| Wasserdampfaufnahme | = 7,6 Prozent |

### Beispiel 2

25 g (0,16 mol) eines hochmolekularen Chitosans, mit einer Grenzviskositätszahl (η) von 1600 ml/g und einem Entacetylierungsgrad von 76 Prozent, werden unter Zusatz einer äquimolaren Menge Sanzlsäure in Wasser gelöst und anschließend durch Einstellen des pH-Wertes auf 8 bis 10 mit Hilfe von Natronlauge wider ausgefällt.

Der voluminöse, über eine große Oberfläche verfügende Niederschlag wird durch mehrmaliges Behandeln mit Wasser neutral gewaschen und also feuchter Filterkuchen in einem Gemisch von 100 ml Wasser und 200 ml Isopropanol dispergiert, mit 55,76 g (0,96 mol) Propylenoxid versetzt und in einem

Druckgefäß unter Rühren bei 100°C 6 Stunden lang zur Reaktion gebracht. Die Aufarbeitung erfolgt wie in Beispiel 1 beschrieben.

Die Ausbeute an N-Hydroxypropyl-chitosan beträgt 31 g.

Kenndaten:

| | |
|---|---|
| Grenzviskositätszahl (η) | = 580 ml/g |
| Substitutionsgrad Hydroxypropyl | = 1,4 |
| Pendelhärte | = 180 sec |
| Wasserdampfaufnahme | = 6,0 Prozent |

Beispiel 3

50 g (0,31 mol) niedermolekulares Chitosan wie in Beispiel 1 werden in einem Gemisch von 100 ml Ethanol und 100 ml Wasser dispergiert und in einer Autoklavenreaktion bei 80°C mit 104,5 g (1,8 mol) Propylenoxid 3 Stunden lang umgesetzt. Die Aufarbeitung erfolgt wie in Beispiel 1 beschreiben.

Es werden 68 g N-Hydroxypropyl-chitosan erhalten.

Kenndaten:

| | |
|---|---|
| Grenzviskositätszahl (η) | = 93 ml/g |
| Substitutionsgrad Hydroxypropyl | = 1,3 |
| Pendelhärte | = 204 sec |
| Wasserdampfaufnahme | = 7,7 Prozent |

Beispiel 4

80,85 g (0,5 mol) niedermolekulares Chitosan wie in Beispiel 1 werden in einem Doppelmantelglasreaktor, der mit Rührer, Thermometer und Rückflußkühler ausgestattet ist, in einem Gemisch aus 415 g (7,15 mol) Propylenoxid und 100 ml Wasser dispergiert. Nach Einstellen des pH-Wertes auf 5 durch tropfenweise Zugabe von konzentrierte Salzsäure, erwärmt man insgesamt 48 Stunden lang auf die Siedetemperatur des Propylenoxids (etwa 35 bis 40°C).

Nach beendeter Reaktion trennt man das Umsetzungsprodukt vom überschüssigen Alkylierungsmittel durch Filtration und wäscht den Rückstand gründlich mit Aceton. Nach Lösen des N-Hydroxypropylchitosans in Wasser wird gegebenenfalls neutralisiert, druckfiltriert und anschließend dialysiert. Die dialysiert, wäßrige Lösung wird am Rotationsverdampfer eingeengt, mit Aceton ausgefällt, auf einer Glassinternutsche gesammelt und bei 50°C im Vakuumtrockenschrank getrocknet.

Man erhält 104 g N-Hydroxypropyl-chitosan.

Kenndaten:

| | |
|---|---|
| Grenzviskositätszahl (η) | = 79 ml/g |
| Substitutionsgrad Hydroxypropyl | = 1,3 |
| Pendelhärte | = 160 sec |
| Wasserdampfaufnahme | = 10,7 Prozent |

Der Substitutionsgrad für die Hydroxypropylreste wurde mit Hilfe der $^{1}H$—NMR-Spektren bestimmt.

Die Messung und Grenzviskositätszahlen erfolgte in einer wäßrigen Lösung von 0,2 mol Essigsäure und 0,1 mol Natriumacetat bei 25°C unter Verwendung eines DIN-Ubbelohde-Viskosimeters.

Die Pendelhärte wurde nach König (W. König, "Härtemessungen mit dem Pendelhärteprüfer", Farbe und Lack *65* (1959), Seite 435 bis 443; DIN 53 137) bestimmt.

Die Wasserdampfaufnahme wurde bei 70 Prozent relativer Luftfeuchte gegenüber 30 Prozent relativer Luftfeuchte ermittelt.

Beispiele für kosmetische Mittel

Beispiel 5 Haarfestiger

| | |
|---|---|
| 0,6 g | N-Hydroxypropyl-chitosan nach Beispiel 1 (η = 62 ml/g, Substitutionsgrad = 1,7) |
| 25,0 g | Isopropanol |
| 0,4 g | Ameisensäure (zehnprozentige, wäßrige Lösung) |
| 0,2 g | Parfümol |
| 73,8 g | Wasser |
| 100,0 g | |

20 ml dieser Lösung werden auf gewaschenes, handtuchtrockenes Haar verteilt. Anschließend werden die Haare in üblicher Weise zur Frisur eingelegt und getrocknet. Bei guter Festigungswirkung zeigt das Haar, im Vergleich zu einem Haarfestiger auf der Basis von Chitosan/Ameisensäure, einen angenehmeren und weicheren Griff.

Beispiel 6 Tönungsfestiger

| | |
|---|---|
| 1,00 g | N-Hydroxypropyl-chitosan nach Beispiel 2 (η = 580 ml/g, Substitutionsgrad = 1,4) |
| 1,00 g | Milchsäure (zehnprozentige, wäßrige Lösung) |
| 0,10 g | Cetyltrimethylammoniumchlorid |
| 0,05 g | Acid Brown 4 (C.I. 14 805) |
| 97,85 g | Wasser |
| 100,00 g | |

20 ml dieser Lösung werden auf dem gewaschenen, handtuchtrockenen Haar verteilt und das Haar in üblicher Weise eingelegt und getrocknet. Das Haar zeigt anschließend eine gute Festigung und eine leichte Rot-Braunfärbung.

Beispiel 7 Tönungsfestiger

| | |
|---|---|
| 0,60 g | N-Hydroxypropyl-chitosan nach Beispiel 3 (η = 93 ml/g, Substitutionsgrad = 1,3) |
| 0,15 g | 1,4-Bis-[(2-hydroxyethyl)-amino]-2-nitro-5-chlor-benzol |
| 25,00 g | Ethanol |
| 74,25 g | Wasser |
| 100,00 g | |

20 ml dieser Lösung werden auf die gewaschenen, handtuchtrockenen Haare gegeben, sodann wird das Haar eingelegt und getrocknet. Die Haare sind rot-violett gefärbt und gefestigt.

Beispiel 8 Anionisches Haarwaschmittel

| | |
|---|---|
| 1,00 g | N-Hydroxypropyl-chitosan nach Beispiel 2 (η = 580 ml/g, Substitutionsgrad = 1,4) |
| 40,00 g | Laurylalkoholdiglykolethersulfat-Natriumsalz (28-prozentige, wäßrige Lösung) |
| 4,00 g | Natriumchlorid |
| 0,05 g | Farbstoff |
| 54,85 g | Wasser |
| 0,10 g | Formaldehyd (25-prozentige, wäßrige Lösung) |
| 100,00 g | |

Es wird ein klares Shampoo erhalten. Das damit gewaschene Haar ist hinsichtlich Griff, Glanz und Kämmbarkeit ausezeichnet konditioniert.

Beispiel 9 Amphoteres, tönendes Haarwaschmittel

| | |
|---|---|
| 2,00 g | N-Hydroxypropyl-chitosan nach Beispiel 1 (η = 62 ml/g, Substitutionsgrad = 1,7) |
| 40,00 g | Dimethyl-carboxymethylen-propylenamidostearat-betain (35-prozentige, wäßrige Lösung) |
| 5,06 g | Ameisensäure (zehnprozentige, wäßrige Lösung) |
| 3,50 g | Kokosfettsäurediethanolamid |
| 1,00 g | Pikraminsäure (einprozentige, wäßrige Lösung) |
| 48,44 g | Wasser, vollentsalzt |
| 100,00 g | |

Mit etwa 15 bis 20 g des obigen Haarwaschmittels wird das Haar einshampooniert. Nach einer Einwirkungszeit von 5 bis 10 Minuten spült man mit Wasser aus. Das Haar ist gelb-orange getönt und ausgezeichnet konditioniert.

Beispiel 10 Katinisches Haarkurmittel

| | |
|---|---|
| 0,30 g | N-Hydroxypropyl-chitosan nach Beispiel 3 (η = 93 ml/g, Substitutionsgrad = 1,3) |
| 4,00 g | Cetylstearylalkohol |
| 1,48 g | Milchsäure (zehnprozentige, wäßrige Lösung) |
| 2,50 g | Kokos(pentaethoxy)methylammoniumchlorid |
| 1,00 g | Sorbitanmonopalmitat, mit 20 Mol Ethylenoxid oxethyliert |
| 90,72 g | Wasser, vollentsalzt |
| 100,00 g | |

35 g des Haarkurmittels nach Beispiel 10 werden im gewaschenen Haar verteilt und nach eine Einwirkungszeit von 3 bis 5 Minuten mit Wasser wieder ausgespült. Als Ergebnis werden ausgezeichneter Griff, Glanz sowie Kämmbarkeit des Haares erhalten.

Beispiel 11 Haarkurmittel, gelförmig

| | |
|---|---|
| 2,1 g | N-Hydroxypropyl-chitosan nach Beispiel 2 (η = 580 ml/g, Substitutionsgrad = 1,4) |
| 0,6 g | Hydroxypropylmethylcellulose |
| 0,5 g | Laurylpyridiniumchlorid |
| 96,8 g | Wasser, vollentsalzt |
| 100,0 g | (auf pH 5,0 mit zehnprozentiger Ameisensäure eingestellt) |

Die Anwendung des Gels erfolgt wie in Beispiel 10 beschrieben wurde. Als Ergebnis werden Griff, Ganz und Kämmbarkeit des Haares wesentlich verbessert.

Beispiel 12 Haartönungsmittel

| | |
|---|---|
| 0,30 g | N-Hydroxypropyl-chitosan nach Beispiel 1 (η = 62 ml/g, Substitutionsgrad = 1,3) |
| 12,00 g | Cetylstearylalkohol |
| 0,10 g | 4-Hydroxy-benzoesäureethylester |
| 6,00 g | Laurylalkoholdiglykolethersulfat-Natriumsalz (28-prozentige, wäßrige Lösung) |
| 0,50 g | Parfümöl |
| 0,50 g | 1-Hydroxy-2-amino-4-nitro-benzol |
| 0,85 g | 1,4-Diamino-2-nitro-benzol |
| 0,24 g | Natriumhydroxid |
| 79,51 g | Wasser |
| 100,00 g | |

30 bis 40 g des Haartönungsmittels werden in dem gewaschenen Haar verteilt und nach einer Einwirkungszeit von etwa 20 Minuten ausgespült. Das Haar ist rötlich gefärbt und weist eine gute Kämmbarkeit und einen angenehmen Griff auf.

Beispiel 13 Oxidationshaarfärbemittel

| | |
|---|---|
| 0,50 g | N-Hydroxypropyl-chitosan nach Beispiel 2 (η = 580 ml/g, Substitutionsgrad = 1,4) |
| 0,08 g | 3,5-Diamino-2,6-dimethoxy-pyridin-dihydrochlorid |
| 0,30 g | 1,4-Diamino-benzol |
| 0,25 g | Resorcin |
| 0,30 g | Natriumsulfit |
| 3,50 g | Laurylalkoholdiglykolethersulfat-Natriumsalz (28-prozentige, wäßrige Lösung) |
| 15,00 g | Cetylalkohol |
| 3,00 g | Ammoniak |
| 77,07 g | Wasser |
| 100,00 g | |

50 g dieses Haarfärbemittels werden mit 50 ml 6-prozentiger Hydrogenperoxidlösung gemischt und auf weißes Haar aufgetragen. Nach 30 Minuten wird das Haar mit Wasser ausgespült und getrocknet. Das Haar hat eine natürlich wirkende matt-blonde Färbung sowie einen natürlichen angenehmen Griff erhalten.

Beispiel 14 Dauerwellmittel

| | |
|---|---|
| 0,5 g | N-Hydroxypropyl-chitosan nach Beispiel 1 (η = 62 ml/g, Substitutionsgrad = 1,7) |
| 10,0 g | Thioglykolsäure |
| 8,0 g | Ammoniak (25-prozentige, wäßrige Lösung) |
| 6,1 g | Ammoniumhydrogencarbonat |
| 75,4 g | Wasser |
| 100,0 g | |

Zur Anwendung trägt man dieses Dauerwellmittel auf das gewickelte handtuchtrockene Haar gleichmäßig auf und läßt es etwa 20 Minuten einwirken. Danach wird das Haar mit Wasser ausgespült und in bekannter Weise oxidativ behandelt. Es wird ein gutes Wellergebnis erhalten, und die Haare fühlen sich natürlich und weich an.

Beispiel 15 Hautcreme

| | |
|---|---|
| 0,30 g | N-Hydroxypropyl-chitosan nach Beispiel 1 (η = 79 ml/g, Substitutionsgrad = 1,3) |
| 3,00 g | Stearylalkohol |
| 1,00 g | Wollwachs (Adeps lanae) |
| 1,00 g | Vaseline |
| 0,76 g | Milchsäure (zehnprozentige, wäßrige Lösung) |
| 1,00 g | Natriumacetylstearylsulfat |
| 92,94 g | Wasser, vollentsalzt |
| 100,00 g | |

Alle Prozentangaben in der vorliegenden Anmeldung stellen Gewichtsprozente dar.

**Patentansprüche**

1. Kosmetisches Mittel zur Behandlung von Haaren oder der Haut, dadurch gekennzeichnet, daß es in einer geeigneten Kosmetikgrundlage ein N-Hydroxypropyl-chitosan, bestehend aus

a) 4 bis 40 Molprozent Einheiten der Formel (I)

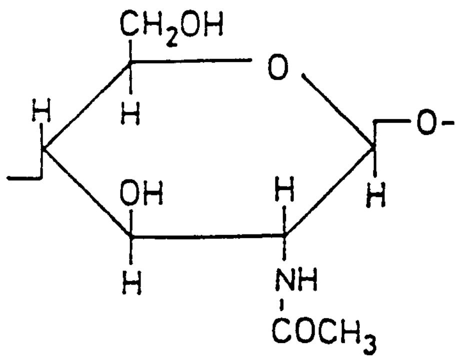

(I)

b) 60 bis 96 Molprozent Einheiten der Formel (II)

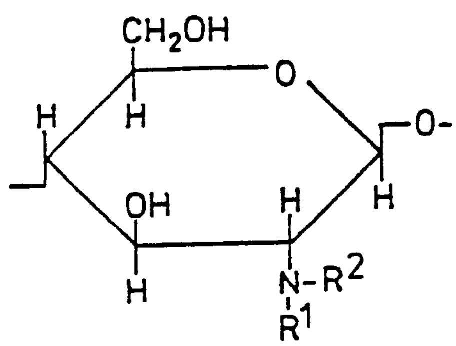

(II),

worin $R^1$ und $R^2$ gleich oder verschieden sind und Wasserstoff oder die Gruppe

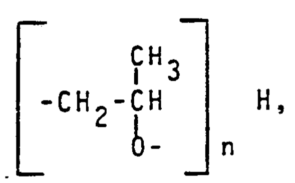

mit n gleich eine ganze Zahl von 1 bis 5, bedeuten unter der Bedingung, daß bei mindestens 50 Prozent der Einheiten der Formel II $R^1$ und $R^2$ nicht gleichzeitig Wasserstoff darstellen, oder dessen Salz enthält.

2. Mittel nach Anspruch 1, dadurch gekennzeichnet, daß es das N-Hydroxypropyl-chitosan in einer Menge von 0,05 bis 10,0 Gewichtsprozent enthält.

3. Mittel nach Anspruch 1 und 2, dadurch gekennzeichnet, daß es in Form einer wäßrigen, alkoholischen oder wäßrig-alkoholischen Zubereitung, insbesondere als Lösung, Creme, Gel, Dispersion oder Emulsion, vorliegt.

4. Mittel nach Anspruch 1 bis 3, dadurch gekennzeichnet, daß der pH-Wert 2 bis 11 beträgt.

5. Mittel nach Anspruch 1 bis 4, dadurch gekennzeichnet, daß es zusätzlich ein bekanntes filmbildendes synthetisches oder natürliches kosmetisches Polymer enthält.

6. Mittel nach Anspruch 1 bis 5, dadurch gekennzeichnet, daß es zisätzlich mindestens einen kosmetischen Farbstoff in einer Konzentration von 0,01 bis 2,0 Gewichtsprozent enthält und in Form eines Farbfestigers oder Tönungsfestigers vorliegt.

7. Mittel nach Anspruch 1 bis 4, dadurch gekennzeichnet, daß es zusätzlich mindestens ein kationisches, nicht-ionisches, amphoteres oder anionisches Tensid enthält und in Form eines Haarwaschmittels vorliegt.

8. Mittel nach Anspruch 7, dadurch gekennzeichnet, daß es das Tensid in einer Konzentration von 3 bis 50 Gewichtsprozent enthält und einen pH-Wert zwischen 3 und 9 aufwest.

9. Mittel nach Anspruch 1 bis 6, dadurch gekennzeichnet, daß es als Kosmetikgrundlage eine wäßrige, alkoholische oder wäßrig-alkoholische Zubereitung enthält, die mit einem unter Druck verflüssigten Treibmittel vermischt ist, in einem Druckbehälter abgefüllt ist und in Form eines Aerosolsprays oder -schaums vorliegt.

10. Verfahren zur Herstellung von makromolekularen, von Chitosan abgeleiteten N-Hydroxypropylverbindungen, bestehend aus

a) 4 bis 40 Molprozent Einheiten der Formel I

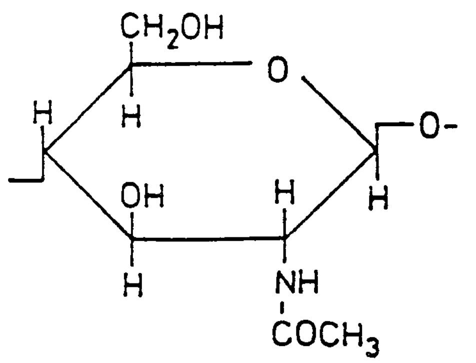

(I)

b) 60 bis 96 Molprozent Einheiten der Formel II

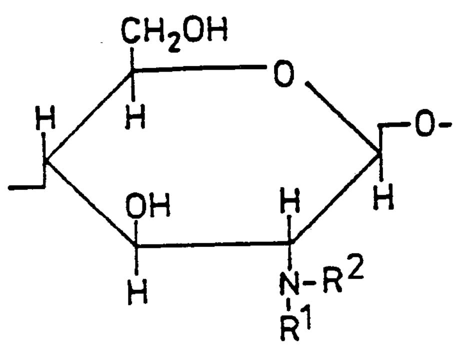

(II),

worin $R^1$ und $R^2$ gleich oder verschieden sind und Wasserstoff oder die Gruppe

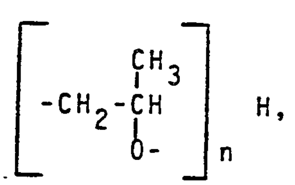

mit n gleich einer ganzen Zahl von 1 bis 5, bedeuten, unter der Bedingung daß bei mindestens 50 Prozent der Einheiten der Formel II $R^1$ und $R^2$ nicht gleichzeitig Wasserstoff darstellen, und deren lösliches Salz mit organischen oder anorganischen Säuren, dadurch gekennzeichnet, daß man ein Chitosan, bestehend aus 60 bis 96 Prozent entacetyliertem Chitin, oder dessen Salz in Gegenwart saurer Katalysatoren in einer Dispersion bestehend aus überschüssigem Propylenoxid und Wasser zur Reaktion bringt.

11. Verfahren nach Anspruch 10, dadurch gekennzeichnet, daß man Chitosan oder dessen Salz mit Propylenoxid bei einer Temperatur von 20 bis 120°C 3 bis 72 Stunden lang umsetzt.

12. Verfahren nach Anspruch 10 und 11, dadurch gekennzeichnet, daß, bezogen auf die eingesetzte Menge Chitosan, das Propylenoxid in einem drei- bis fünfzehnfachen molaren Überschuß eingesetzt wird.

**Revendications**

1. Agent cosmétique pour la traitement des cheveux ou de la peau, caractérisé en ce qu'il contient dans une base de cosmétique un N-hydroxypropyl-chitosane constituée de:

a) 4 à 40 moles pour cent d'unités de formule (I)

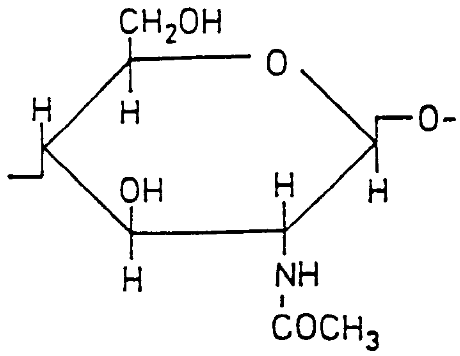

(I)

b) 60 à 90 moles pour cent d'unités de formule (II)

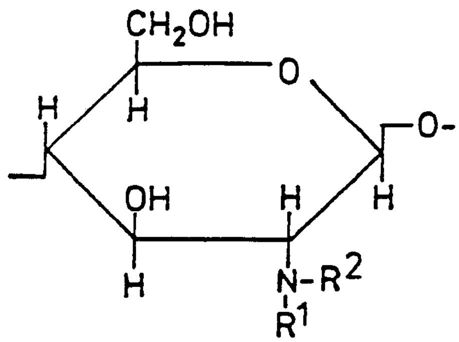

(II),

dans lesquelles $R^1$ et $R^2$, identiques ou différents représentent l'hydrogène ou le groupe

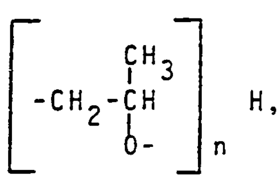

n étant de même un nombre entier allant de 1 à 5, avec la condition qu'au moins 50% des unités de formule II $R^1$ et $R^2$ ne représente pas simultanément l'hydrogène, ou son sel.

2. Agent selon la revendication 1, caractérisé en ce qu'il contient le N-hydroxypropyl-chitosane à raison de 0,05 à 10,0% en poids.

3. Agent selon les revendications 1 et 2, caractérisé en ce qu'il se présente sous forme d'une préparation aqueuse, alcoolique ou hydro-alcoolique, en particulier sous forme de solution, crème, gel, dispersion ou émulsion.

4. Agent selon les revendications 1 à 3, caractérisé en ce que la valeur du pH va de 2 à 11.

5. Agent selon les revendications 1 à 4, caractérisé en ce qu'il contient au moins additionnelement un polymère cosmétique filmogène naturel ou synthétique.

6. Agent selon les revendications 1 à 5, caractérisé en ce qu'il contient en plus un colorant cosmétique à raison de 0,01 à 20% en poids et se présente sous la forme d'un fixateur de couleur ou de nuance.

7. Agent selon les revendications 1 à 4, caractérisé en ce qu'il contient en outre au moins un agent tensioactif cationique, non-ionique, amphothère ou anionique et qu'il se présente sous forme de shampooing.

8. Agent selon la revendication 7, caractérisé en ce qu'il contient l'agent tensio-actif à raison de 3 à 50% en poids et présente une valeur de pH comprise entre 3 et 9.

9. Agent selon les revendications 1 à 6, caractérisé en ce qu'il contient comme base de cosmétique une préparation aqueuse, alcoolique ou hydro-alcoolique qui est mélangée à un agent propulseur liquéfié sous pression, chargée dans un récipient sous pression et se présente sous la forme d'une pulvérisation d'aérosol ou de mousse.

10. Procédé de préparation des composés macromoléculaires, de N-hydroxypropyle dérivé du chitosane, se composant de:

a) 4 à 40 moles pour cent d'unités de formule

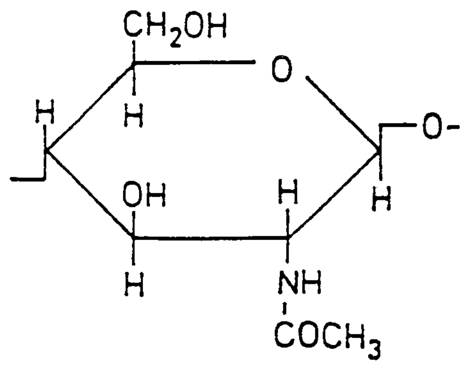

(I)

b) 60 à 90 moles pour cent d'unités de formule (II)

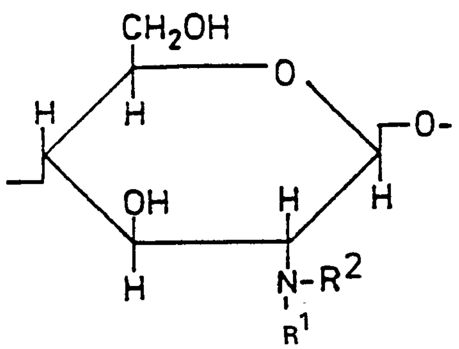

(II),

dans laquelle $R^1$ et $R^2$ identique ou différents sont soit l'hydrogène soit le groupe:

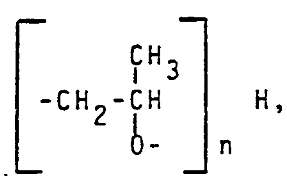

n étant de même un nombre entier allant de 1 à 5, avec la condition qu'au moins 50% des unités de formule II $R^1$ et $R^2$ ne représentent pas simultanément l'hydrogène, et leurs sels solubles d'acides organique ou minéraux, caractérisé en ce qu'on fait réagir un chitosane, constitué de 60 à 96% de chitine desacétylée, ou son sel en présence de catalyseurs acides dans une dispersion constituée d'oxyde de propylène en excés et d'eau.

11. Procédé selon la revendication 10, caractérisé en ce qu'on fait réagir le chitosane ou son sel à une température de 20 à 120°C pendant 3 à 72 heures.

12. Procédé selon les revendications 10 et 11, caractérisé en ce qu'on incorpore l'oxyde de propylène dans un excés molaire de trois à quinze fois par rapport à la quantité de chitosane utilisée.

**Claims**

1. Cosmetic composition for treating hair or skin, characterised in that it contains in a suitable cosmetic base an N-hydroxypropyl chitosan, consisting of

a) 4 to 40 mol% monomer units of the formula (I),

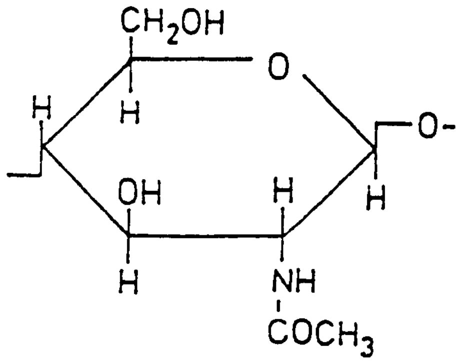

(I)

and

b) 60 to 96 mol% monomer units of formula (II)

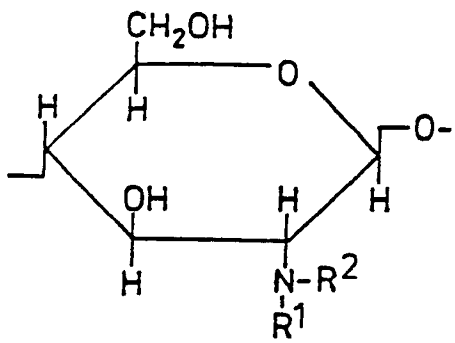

(II),

in which $R^1$ and $R^2$ are equal or different and represent either hydrogen or the group

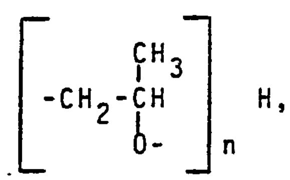

with n being equal to an integer from 1 to 5, provided that in the case of at least 50% of the units of formula (II) $R^1$ and $R^2$ do not represent hydrogen at the same time, or its soluble salt.

2. Composition according to claim 1, characterised in that the N-hydroxypropyl chitosan is contained in a quantity of 0.05 to 10.0% by weight.

3. Composition according to claims 1 and 2, characterised in that it is present in the form of an aqueous, alcoholic or aqueous-alcoholic preparation, in particular as a solution, cream, gel, dispersion or emulsion.

4. Composition according to claims 1 to 3, characterised in that the pH value is 2 to 11.

5. Composition according to claims 1 to 4, characterised in that it contains in addition a known film-forming synthetic or nature cosmetic polymer.

6. Composition according to claims 1 to 5, characterised in that it contains in addition at least a cosmetic colouring matter in a concentration of 0.01 to 2.0% by weight and is present in the form of a colour strengthener or tinting strengthener.

7. Composition according to claims 1 to 4, characterised in that it contains in addition one cationic, nonionic, amphoteric or anionic tenside and is present in the form of a shampoo.

8. Composition according to claim 7, characterised in that it contains the tenside in a concentration of 3 to 50% by weight and has a pH value of between 3 and 9.

9. Composition according to claims 1 to 6, characterised in that it contains an aqueous, alcoholic or aqueous-alcoholic preparation as a cosmetic base, which is mixed with a propellant liquified under pressure, filled into a pressure container and is present in the form of an aerosol spray or foam.

10. Process for producing macromolecular N-hydroxypropyl compounds derived from chitosan, comprising

a) 4 to 40 mol% monomer units of the formula (I)

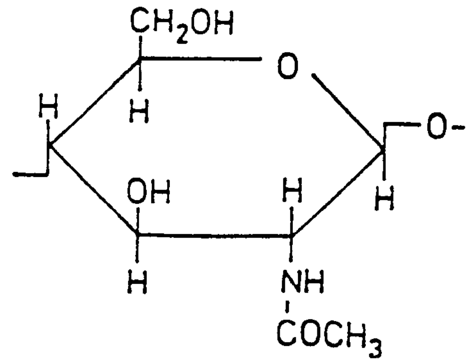

(I)

and

b) 60 to 96 mol% monomer units of formula (II)

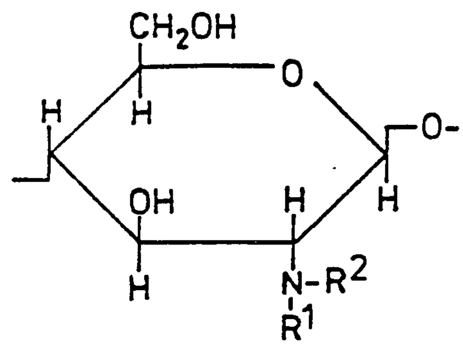

(II),

in which $R^1$ and $R^2$ are equal or different and represent either hydrogen or the group

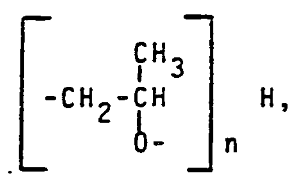

in which n is equal to an integer from 1 to 5, provided that in the case of at least 50% of the units of formula (II) $R^1$ and $R^2$ do not represent hydrogen at the same time, or their soluble salts with organic or inorganic acids, characterised in that chitosan, consisting of 60 to 96% deacetylated chitin, or its salt is reacted in the presence of acid catalysts in a dispersion consisting of excess propylene oxide and water.

11. Process according to claim 10, characterised in that chitosan or its salt is reacted with propylene oxide at a temperature of from 20 to 120°C for e to 72 hours.

12. Process according to claims 10 and 11, characterised in that the propylene oxide is employed in a three to fifteenfold excess with regard to the quantity of chitosan used.